(19) Europäisches Patentamt
European Patent Office
Office européen des brevets

(11) **EP 3 794 320 B1**

(12) **EUROPEAN PATENT SPECIFICATION**

(45) Date of publication and mention
of the grant of the patent:
**13.12.2023 Bulletin 2023/50**

(21) Application number: **19734397.3**

(22) Date of filing: **28.06.2019**

(51) International Patent Classification (IPC):
**G01F 1/74** *(2006.01)*     **G01N 27/74** *(2006.01)*
**G01N 33/28** *(2006.01)*     G01F 1/58 *(2006.01)*
G01F 1/712 *(2006.01)*     G01V 3/28 *(2006.01)*

(52) Cooperative Patent Classification (CPC):
**G01F 1/74; G01N 27/74; G01N 33/2823;**
G01F 1/58; G01F 1/582; G01F 1/712; G01V 3/28

(86) International application number:
**PCT/EP2019/067422**

(87) International publication number:
**WO 2020/002647 (02.01.2020 Gazette 2020/01)**

(54) **METHOD AND APPARATUS FOR MONITORING OF THE MULTIPHASE FLOW IN A PIPE**

VERFAHREN UND VORRICHTUNG ZUR ÜBERWACHUNG DER MEHRPHASENSTRÖMUNG IN
EINEM ROHR

PROCÉDÉ ET APPAREIL DE SURVEILLANCE D'UN ÉCOULEMENT POLYPHASIQUE DANS UN
TUYAU

(84) Designated Contracting States:
**AL AT BE BG CH CY CZ DE DK EE ES FI FR GR
HR HU IE IS IT LI LT LU LV MC MK MT NL NO PL
PT RO RS SE SI SK SM TR**

(30) Priority: **29.06.2018 GB 201810733**

(43) Date of publication of application:
**24.03.2021 Bulletin 2021/12**

(73) Proprietor: **Flodatix Limited
Basingstoke
Hampshire RG24 8NE (GB)**

(72) Inventors:
• **MA, Lu
Basingstoke, Hampshire RG24 8NE (GB)**

• **ARELLANO, Yessica
Basingstoke, Hampshire RG24 8NE (GB)**

(74) Representative: **Page White Farrer
Bedford House
21a John Street
London WC1N 2BF (GB)**

(56) References cited:
WO-A1-02/079770     CN-A- 101 871 906
GB-A- 2 530 601     US-A1- 2018 325 414

• PEYTON A J ET AL: "Development of
electromagnetic tomography (EMT) for industrial
applications. Part 1: sensor design and
instrumentation", 19990414, 14 April 1999
(1999-04-14), pages 306-312, XP002976895,

## Description

The present invention relates to a method of, and a monitoring apparatus for, monitoring a multiphase flow in a pipe using magnetic induction tomography. The multiphase flow comprises fluids, and may comprise a mixture of liquids, or one or more liquids in a mixture with solids and/or gases. This invention relates to a multiphase flow metering apparatus and method which has a number of applications, in particular within the oil and gas exploration and production industry.

A number of prior patent specifications in the name of the Applicant are directed to the use of Magnetic Induction Tomography (MIT), either used alone or in conjunction with other techniques, for monitoring a multiphase flow in a pipe, in particular in the oil and gas exploration and production industry.

In particular, GB2513678B discloses an "Oil well system and operating method including monitoring multiphase flow in a pipe", GB2513679B discloses a "Method of defining a multiphase flow comprising three phases", GB2507368B discloses "Method and apparatus for monitoring the flow of mixtures of fluids in a pipe", GB2534337B discloses "Method and apparatus for monitoring of the multiphase flow in a pipe" and GB2530601B discloses "Method and apparatus for monitoring of the multiphase flow in a pipe".

In these prior specifications, helical and circular transmitting and receiving coils are located around the outside of a pipe. In this specification, the term "helical" means a non-planar spiral in three dimensions, i.e. having the shape of a geometrical helix. The transmitting coil(s) are supplied with a varying current which transmits electromagnetic field into a multiphase flow within the pipe. The electromagnetic field induces eddy currents in a conductive phase within the pipe resulting in a secondary electromagnetic field. The secondary electromagnetic field induces an electric field which can be measured in the receiving coil(s) via voltage output that can be analysed to determine properties of the multiphase flow.

In addition, GB2527324B discloses a "Segmented Electromagnetic Sensor".

It is disclosed is these specifications that electromagnetic energy can provide information related to certain physical properties of materials in the multiphase flow exposed to this type of energy. When used in an electromagnetic flowmeter, electrical capacitance tomography (ECT), electrical resistance tomography (ERT) and magnetic inductance tomography (MIT) can be used to interrogate the multiphase flow. In each case a varying electric or magnetic field can be applied across the multiphase flow, and measurements of voltage, current and magnetic field can be used to measure certain physical parameters of the constituent components of the multiphase flow.

Apart from these prior patent specifications and other disclosure by the present Applicant or its employees or the present inventors, although MIT is known for use in various medical applications, the present inventors are not aware of any other disclosure in the context of providing real time data on oil/gas/water multiphase flow imaging, for example for use in the oil and gas industry. It is known from such medical applications to use various coil geometry developments for static or semi-static fluid tests using helical coils with various numbers of turns. However, no optimum coil performance for flow measurement has been defined.

The behaviour of helical coils are not characterised in the current literature, although several variants of circular helical coils are disclosed, in particular having a balanced coil configuration, a particular number of turns for the transmitting and receiving coils, various types of wire configurations (i.e. insulated wires or stranded wires), which are for deployment in a static or quasi-static or phantom simulated 2- phase or 3-phase flow in a laboratory setting.

CN 101871906 A discloses an apparatus for multiphase fluid imaging based on double-modal tomography comprising a pumping signal generating unit, sensor array, signal condition and collecting unit and a host computer. The sensor array comprises at least two stimulus sensors and at least two receiving sensors that are distributed on the outside of a pipe on the same cross-section, wherein the stimulus sensor is a plate coil and the receiving sensor comprises a dash receiver and a coupled plate coil. The signal generated by the pumping signal generating unit is transmitted by the stimulus sensor, the receiving sensor receives a signal and sends the signal to the host computer.

Although the patent specifications identified above disclose a method of, and a monitoring apparatus for, monitoring a multiphase flow in a pipe using magnetic induction tomography which can effectively monitor such a multiphase flow, there is nevertheless a need in the art for an improved monitoring apparatus and method.

In particular, there is a need in the art for a monitoring apparatus and method which has an enhanced sensitivity to distinguish between an electrically conductive phase such as an aqueous phase, for example water, and an electrically non-conductive phase, such as an oil phase, in a multiphase flow.

The present invention aims, at least partially, to meet this need in the art, particularly in the field of oil and gas exploration and production, to provide enhanced analytical data in real-time on the phase composition of multiphase fluid/solid flows particularly within a pipeline used in the oil and gas industry.

The present invention accordingly provides a monitoring apparatus for monitoring a multiphase flow in a pipe using magnetic induction tomography according to claim 1.

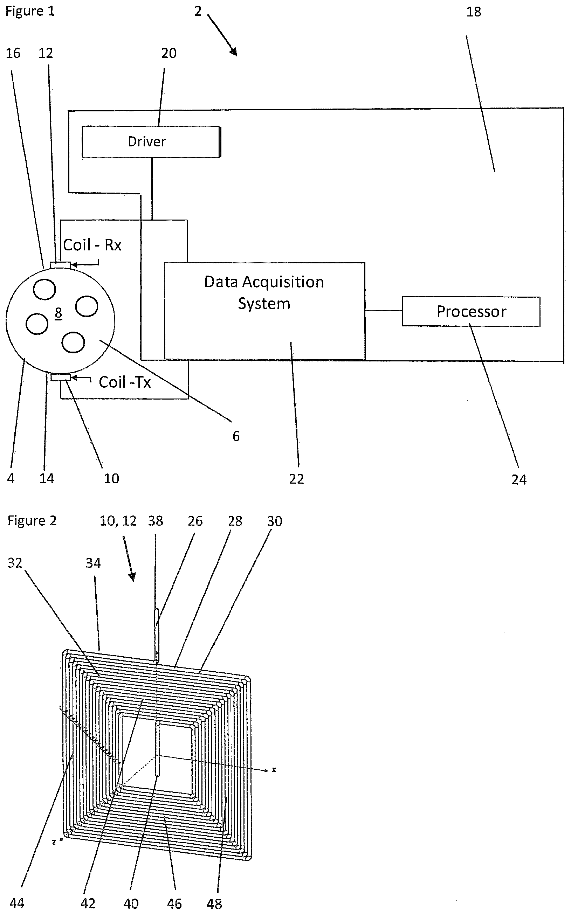

In this specification, the term "planar" encompasses an element having a curvature so as to be oriented in a curved direction associated with and around the corresponding curvature of the circumference of the pipe. However, in the preferred embodiments of the present invention each turn is in a common plane that is geometrically planar so that the respective coil is geometrically planar, for example as illustrated in Figure 2 described hereinafter.

In some preferred embodiments, each turn is composed of four linear sections and the respective coil is rectangular in plan. Typically, the rectangular coil has a length: width ratio of from 1:1 to 3:1, optionally from 1:1 to 2:1.

In some preferred embodiments, the linear sections are equal in length and the respective coil is a regular polygon in plan. In one particularly preferred embodiment, the respective coil is square in plan.

In one embodiment, the first and second coils are disposed on respective diametrically opposite first and second sides of the pipe. The central axis of the first coil may be coaxial with the central axis of the second coil. However, the first and second coils may alternatively not be directly opposite, but may be at any angle or orientation so that an electromagnetic field signal transmitted from one coil into the multiphase flow in the conduit can cause a secondary electromagnetic field signal from the multiphase flow to be received by the other coil.

However, particularly preferred embodiments of the monitoring apparatus of the present invention incorporate a set of a plurality of coils, typically 8 coils, disposed as one or more annular arrays around the pipe in one or more planes.

In the first and second coils, the electrically conductive element comprises an electrically conductive wire core surrounded by an electrically non-conductive outer layer. Typically, the electrically conductive core is composed of copper. Preferably, the core has a diameter of from 0.2 to 1 mm, optionally from 0.25 to 0.75 mm. Typically, the electrically non-conductive outer layer is composed of an enamel. Preferably, the enamel outer layer has a thickness 0.02 to 0.04 mm.

In the first and second coils, successive turns are mutually adjacent and in contact, and adjacent turns of the electrically conductive element are mutually separated by an electrically insulating material. Preferably, in the first and second coils, successive turns are mutually adjacent and in contact along the entire length of each turn.

In some preferred embodiments, each of the first and second coils has from 10 to 30 turns, for example from 15 to 25 turns. Typically, each of the first and second coils has an external dimension, in the plane of the respective coil, within the range of from 20 to 50 mm, for example from 25 to 45 mm. Typically, each of the first and second coils has an external dimension, in the plane of the respective coil relative to a diameter of the pipe within the range of from 0.1D to 0.8D, for example from 0.2D to 0.4D.

Preferably, the monitoring apparatus further comprises a controller for selectively switching alternating electrical current through one of the first and second coils to generate from the respective energized coil a local electromagnetic field which is transmitted into the fluid conduit, and for receiving an induced alternating electrical current from the other of the first and second coils which is generated from the local electromagnetic field.

The present invention further provides a method of monitoring a multiphase flow in a pipe using magnetic induction tomography according to claim 9.

Preferably, in step c alternating electrical current having a frequency of from 1 kHz to 30 MHz, optionally from 1 kHz to 10 MHz, is switched through the first coil to generate from the respective energized coil a local electromagnetic field which is transmitted into the fluid conduit.

The multiphase flow may comprise at least two phases, optionally at least three phases, further optionally all of the phases, selected from an oil phase, an aqueous phase, a solid phase and a gaseous phase.

When monitoring a multiphase flow in a pipe using magnetic induction tomography, when the multiphase flow includes an electrically conductive phase, such as an aqueous phase, for example water, various parameters may be relevant to define the performance and accuracy of the monitoring.

The present invention is at least partly predicated on the finding by the present inventors that when each of the first and second coils comprises an electrically conductive element which is wound about a central axis to form a plurality of turns, wherein each turn is in a common plane so that the respective coil is planar and each turn is composed of a plurality of linear sections and the respective coil is polygonal in plan, the monitoring apparatus and method can achieve a higher phase sensitivity, a larger phase dynamic range and a greater induced eddy current in a region of interest of a pipe that contains a multiphase flow, as compared to the use of other coil configurations, as disclosed in the state of the art. By achieving these enhanced parameters, the accuracy of the measurement and monitoring of the multiphase flow can be improved.

As described above, the known MIT systems tend to implement circular helical coils, which is believed to result from ease of construction and low cost of the manufacturing process, which is usually a manual manufacturing process.

It is to be noted that the Applicant's earlier GB2530601B, entitled, "Method and apparatus for monitoring of the multiphase flow in a pipe", discloses helical and circular transmitting and receiving coils. In one embodiment, described with reference to Figure 7, an electromagnetic "intelligent" screen array is provided to function as a screening device for screening one or more of the helical and circular transmitting or receiving coils from an interfering electromagnetic field emitted from one or more other helical and circular transmitting or receiving coils, the screening device comprising an annular screen located around the pipe. Although the electromagnetic "intelligent" screen array may comprise an array of plural planar square coil elements, there is no disclosure or suggestion to modify the coil configuration of the helical and circular transmitting or receiving coils.

In particular, the present inventors have found that by changing the coil geometry of the transmitting and receiving coils, the known monitoring apparatus and method can be further enhanced, in terms of the phase dynamic range, phase sensitivity, and induced eddy current intensity, in the region of interest and hence can achieve an enhanced measurement accuracy of MIT.

The following terms are defined to describe the advantages of the coil configurations of the present invention.

The phase measurement ($\varphi$ in rad) is defined by the phase angle of the induced voltage from a receiving coil relative to a reference due to the induced eddy currents ($J_{eddy}$) around conductive medium in the region of interest.

The phase difference ($\Delta\varphi$ in rad) is defined by the difference between the phase angles of the induced voltage from a coil due to a change in conductivity arising from change in medium structure, motion, an external environment (in either the spatial or time domains), as well as frequency-dependent characteristics.

The phase sensitivity $\varphi_s$ is defined by phase difference per unit conductivity change, i.e.; $\varphi_s =$

$$\frac{\Delta\varphi}{\Delta\sigma} \ (rad/Sm^{-1})$$

for a given water volume flowrate fraction.

The phase dynamic range $\varphi_r = [\varphi_{VF_w=0}, \varphi_{VF_w=1}]$ is defined by the phase measured at water volume flowrate fraction from 0 to 1.

The eddy current range $J_{eddy} = [J_{VF_w=0}, J_{VF_w=1}]$ is defined by the norm component of induced eddy currents in the region of interest at water volume flowrate fraction from 0 to 1.

In these parameters, the water volume flowrate

$$F_W = \frac{Q_w}{Q_w + Q_g + Q_o}$$

fraction is defined by                    , and $Q_w$, $Q_o$, $Q_g$ are the volumetric flowrate of water, oil and gas respectively.

The present inventors unexpectedly found that by using the coil geometry of the present invention, which is a coil design that departs from traditional helical coil geometries, and in contrast is a coil formed by a plurality of concentric conductive turns to form a polygonal, non-helical, non-circular, coil with turns which are arranged in a single plane, and preferably with there being with no air gap between adjacent turns of the planar, polygonal coil, the resultant monitoring apparatus and method can achieve an increased phase dynamic range, an increased phase sensitivity and increased induced eddy currents in the cross-section of the region of interest of the multiphase flow as compared to the use of the known monitoring apparatus and methods using known coil structures, in particular helical coils.

Furthermore, the present inventors have found that by using the coil geometry of the present invention, the number of turns of the coils can be increased, to enhance measuring sensitivity and accuracy, without increasing the radial dimensions of the monitoring system. The present inventors have found unexpectedly that there can be an increase, with increasing number of turns, in the phase dynamic range of the planar geometry, whereas in contrast it is known in the prior art from the behaviour of helical coils that there tends to be a decreasing performance of measurement accuracy and sensitivity with an increasing number of coil turns.

In accordance with the present invention, in a monitoring apparatus and method for measuring multiphase flow in a pipe, a planar, polygonal coil design is used for transmitting and receiving coils that departs from conventional helical, circular coil geometries. The planar, polygonal coil is formed by a plurality of concentric conductive turns, preferably with no air gap between the adjacent turns, which are arranged in a single plane. By avoiding any physical air gap between adjacent turns, in each coil the maximum number of turns can be achieved in any given XY space around the pipe perimeter, so that any potentially detrimental effect of spatial constraints is minimised or avoided without compromising the phase monitoring sensitivity.

The present invention relates specifically to an improved apparatus and method for the use of MIT (Magnetic Induction Tomography), in particular in the application of MIT to measuring multiphase flows in the oil and gas and other industries. The principle of MIT is that electric coils are excited with alternating current that results in the coils producing varying electromagnetic fields. The object of interest is placed within these fields and the varying field induces varying currents within the object that is dependent on the conductivity of the object. The varying currents in the object produce secondary electromagnetic fields that can be received by the same or other coils. The received secondary electromagnetic field in conjunction with the primary imposed electromagnetic field can be used to compute the conductivity contrast between the object and the material that surrounds it.

The preferred embodiments of this invention relate to an apparatus and method to measure the flow of mixtures of fluids from a well or group of wells during oil and gas exploration, production or transportation operations. However, it should be understood that the apparatus and method of the present invention may be used in other potential applications, as those skilled in the art will appreciate. For example, the apparatus and method of the present invention may be used in flow measurement devices, medical MIT Systems involving measurement of low conductivity contrasts of multiphase fluid flows, and multiphase process monitoring equipment.

Embodiments of the present invention will now be described, by way of example only, with reference to the accompanying drawings, in which:

Figure 1 is a schematic illustration of a monitoring apparatus for monitoring a multiphase flow through a pipe in accordance with an embodiment of the present invention;

Figure 2 is an enlarged schematic perspective view of a coil for transmitting or receiving electromagnetic radiation in the apparatus of Figure 1;

Figure 3 is an enlarged schematic cross-section through a wire of the coil for transmitting or receiving electromagnetic radiation in the apparatus of Figure 1;

Figure 4 is a graph showing the relationship be between normalised phase sensitivity and number of turns for a planar polygonal coil for use in the apparatus of Figure 1 and for two alternative known coil configurations used in the state of the art;

Figure 5 is a graph showing the relationship between normalised phase dynamic range and number of turns for a planar polygonal coil for use in the apparatus of Figure 1 and for two alternative known coil configurations used in the state of the art; and

Figure 6 is a graph showing the relationship between normalised eddy current range and number of turns for a planar polygonal coil for use in the apparatus of Figure 1 and for two alternative known coil configurations used in the state of the art.

Referring to Figures 1 to 3, there is shown a monitoring apparatus 2 for monitoring a multiphase flow in a pipe using magnetic induction tomography.

The apparatus 2 comprises a pipe 4 defining a flow conduit 6. In use, a multiphase flow 8 flows along the flow conduit 6 of the pipe 4. The multiphase flow 8 comprises at least two phases, optionally at least three phases, further optionally all of the phases, selected from an oil phase, an aqueous phase, a solid phase and a gaseous phase. In Figure 1, two phases are shown schematically, comprising a continuous phase and a discontinuous phase. The multiphase flow 8 typically has a primary or continuous phase of the flow, e.g., oil, water or gas, and within the primary phase one or more other phase constituents may be present, for example a solid phase, e.g. sand. The flow regime of these phases can vary significantly depending on the concentrations of each phase and the flow rate.

A first coil 10 and a second coil 12 are disposed on respective opposite first and second sides 14, 16 of the pipe 4, which are typically diametrically opposite first and second sides 14, 16 of the pipe 4. Preferably, a central axis, shown as axis Z in Figure 2, of the first coil 10 is coaxial with a central axis of the second coil 12.

The portion of the pipe 4 between the first and second coils 10, 12 is preferably composed of a material, for example a non-metallic material such as a polymer, which permits electromagnetic field penetration, across a broad wavelength range, emitted from one of the first and second coils 10, 12 to pass through the multiphase material in the flow conduit 6 and then secondary induced electromagnetic radiation to be received by the other of the first and second coils 10, 12.

In the illustrated embodiment, the first coil 10 is configured to function as a transmitter coil, indicated by Tx, and the second coil 12 is configured to function as a receiver coil, indicated by Rx. However, each of the first and second coils 10, 12 may be adapted to transmit an electromagnetic field into the flow conduit 6 when energized by an input electrical signal and/or to receive an electromagnetic field from the flow conduit 6 and generate an output electrical signal, as described in detail hereinafter. In one embodiment the first coil 10 is always a transmitter and the second coil 12 is always a receiver; in other embodiments the first coil 10 and the second coil 12 alternate between functioning as the transmitter and as the receiver. Also, there can be more than two coils around the pipe 4. Fundamentally, any arrangement or control of the first and second coils 10, 12 may be employed which can transmit an electromagnetic field into multiphase flow 8 in the flow conduit 6 and receive a resultant electromagnetic field from the multiphase flow 8 in the flow conduit 6, and thereby generate an output electrical signal to be used to analyse the phase composition of the multiphase flow 8 using magnetic induction tomography.

The first coil 10 and second coil 12 are electrically connected to a controller 18. The controller 18 comprises a driver 20 for providing alternating electrical current, i.e. AC current, at a selected frequency to the transmitter coil, for example the first coil 10 as in the illustrated embodiment. Typically, the AC current has a frequency of from 1 kHz to 30 MHz, for example from 1 kHz to 10 MHz.

The controller 18 also comprises a data acquisition system 22, coupled to a processor 24. The data acquisition system 22 receives the induced electrical current from the receiver coil, for example the second coil 12 as in the illustrated embodiment, and the current is analysed, by use of the processor 24, to provide an output signal which can provide, or be further analysed to provide, a phase difference measurement, of the multiphase flow, by magnetic induction tomography.

In accordance with the present invention, each of the first and second coils 10, 12 has a specific structure which has unexpectedly been found to provide an enhanced analysis of the multiphase flow by magnetic induction tomography as compared to known coil designs utilized in the prior art as discussed hereinabove.

Accordingly, referring to Figure 2 which shows the structure for each of the first and second coils 10, 12, each of the first and second coils 10, 12 comprises an electrically conductive element 26 in the form of a wire 26 which is wound about a central axis Z to form a plurality of wire turns 28. Each turn 28 is in a common plane XY so that the respective coil 10, 12 is planar. Furthermore, each turn 28 is composed of a plurality of linear sections 30 so that the respective coil 10, 12 is polygonal in plan. Therefore the wire 26 extends in a spiral 32 between outer and inner peripheral edges 34, 36 of the respective coil 10, 12. The wire 26 has opposite outer and inner ends 38, 40 that are electrically connected to the controller 18. In the illustrated embodiment, each turn 28 is composed of four linear sections 42, 44, 46, 48 and the respective coil is rectangular in plan. Typically, the rectangular coil 10, 12 has a length: width ratio of from 1:1 to 3:1, optionally from 1:1 to 2:1. In the illustrated embodiment, the linear sections 42, 44, 46, 48 are equal in length and the respective coil 10, 12 is square in plan. In other embodiments, the linear sections are equal in length and the respective coil is a regular polygon in plan, and may, for example have more than four sides, for example five or six sides.

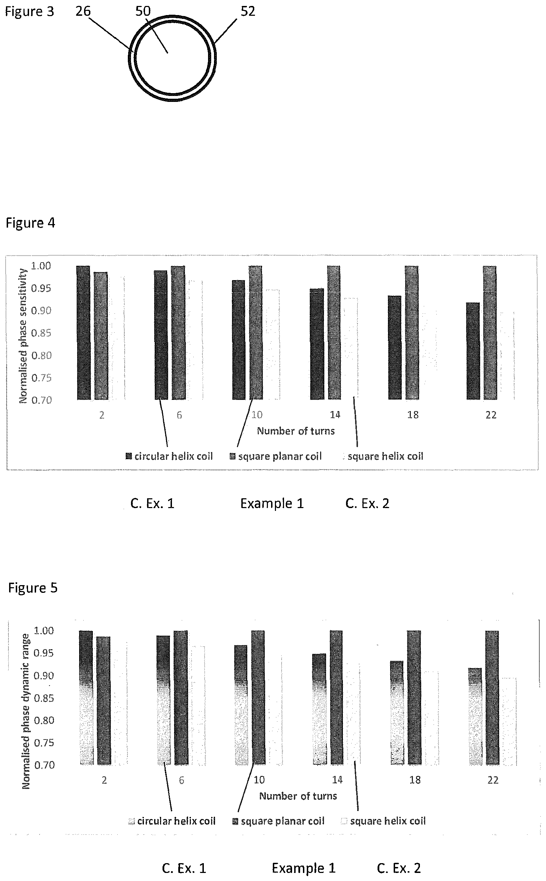

Referring to Figure 3, in the first and second coils 10, 12, the electrically conductive wire 26 comprises an electrically conductive core 50 surrounded by an electrically non-conductive outer layer 52. Typically, the electrically conductive core 50 is composed of copper. Optionally, the copper core 52 has a diameter of from 0.2 to 1 mm, for example from 0.25 to 0.75 mm, for example about 0.5 mm. Typically, the electrically non-conductive outer layer 52 is composed of an enamel. Optionally, the enamel outer layer 52 has a thickness 0.02 to 0.04 mm, for example about 0.03 mm.

In the first and second coils 10, 12, successive turns 28 are mutually adjacent and in contact, and more preferably in contact along the entire length of each turn 28. When such contact is present, adjacent turns 28 of the electrically conductive element 26 are mutually separated by an electrically insulating material. This provides that there is no gap between the adjacent turns 28 of the coil 10, 12. Typically, each of the first and second coils 10, 12 has from 10 to 30 turns, optionally from 15 to 25 turns, for example 18 turns. Typically, each of the first and second coils 10, 12 has an external dimension, in the plane of the respective coil 10, 12, within the range of from 20 to 50 mm, optionally from 25 to 45 mm.

Typically, each of the first and second coils 10, 12 has an external dimension, in the plane of the respective coil 10, 12, relative to a diameter (D) of the pipe 4 within the range of from 0.1D to 0.8D, for example from 0.2D to 0.4D.

In the method of monitoring a multiphase flow in a pipe using magnetic induction tomography, there is provided a pipe 4 defining the flow conduit 6, and the first and second coils 10, 12 disposed on the respective opposite first and second sides 14, 16 of the pipe 4 but those skilled in the art will appreciate that more coils could be used to provide more measurements across the pipe 4.

An electromagnetic field is transmitted from the first coil 12 into the multiphase flow 8. The multiphase flow 8 may comprise any combination of two or more of an oil phase, an aqueous phase, a solid phase and a gaseous phase. The multiphase flow 8, when the invention is being used in the oil industry, typically comprises an oil phase, and an aqueous phase, and optionally one or both of a solid phase and a gaseous phase.

The second coil 12 receives an electromagnetic field from the eddy currents induced in the multiphase flow 8 and generates an output electrical signal therefrom. Each of the first and second coils 10, 12 can act as either a transmitting or receiving coil and can change between the two transmitting and receiving modes. As described above, an alternating electrical current, preferably having a frequency of from 1 kHz to 10 MHz, is switched through the first coil 10 to generate from the respective energized coil 10 a local electromagnetic field which is transmitted into the fluid conduit 6.

Thus a varying electric current is passed through the first coil 10, which may have the properties of a sine wave, or another form, e.g. square wave, and all other potential forms of varying current are encompassed by the present invention. The varying electric current passing through the first coil 10 generates a varying electromagnetic flux through the multiphase flow 8 that is within the pipe 4. Depending on the physical properties of the different phases that the electromagnetic flux lines interrogate and in particular the electrical conductivity contrast between the phases, for example the oil and aqueous phases, a varying current is induced in any electrically conductive phase. This induced current in turn generates a secondary varying electromagnetic field that propagates through the pipe 4 and is picked up by the second coil 12 that is used as a receiver. The secondary varying electromagnetic field therefore induces a varying current in the receiver coil 12. By comparing the driving and induced currents using appropriate processing, for example, the phase shift between the signals, allows the conductivity contrast between the materials of the multiphase flow to be computed.

The illustrated embodiment comprises two coils, namely the first and second coils 10, 12, which can function in a fixed operating mode, i.e. the first coil 10 acts as a transmitting coil and the second coil 12 acts as a receiving coil, or in a dynamic operating mode, i.e. the first and second coils 10, 12 can be switched so as to alternate between functioning as a transmitting coil and as a receiving coil.

In further embodiments, additional coils are provided, which have the same planar and polygonal coil structure as described above for the first and second coils 10, 12. When more than two coils are provided, typically one coil may function as a transmitter and other coils may function as a receiver, so that, for example, at any point in time there is one coil that is transmitting and all of the other coils are receiving. Once all the receiver coil signals have been processed, one or more of the other coils becomes the transmitter and again the remainder are receivers and so forth.

It will be appreciated by those skilled in the art that the sequencing may take place in any order and that a complete cycle of measurements, that is, where every coil has been the transmitter once, can occur very rapidly with, e.g., 160 to 8000, or 500 to 5000, measurement cycles every second. This frequency being primarily limited only by the processing power of the processor 24. It will also be appreciated by those skilled in the art that after one complete cycle of measurements a mesh of properties is produced that can be processed to provide a mesh or image of the multiple phases across the section of the pipe.

Although this description describes each coil being either a transmitter or receiver, clearly, a configuration can be provided whereby certain coils are always transmitters and others are always receivers. In other embodiments coils can be enclosed within other coils so that dedicated transmitter and receiver coils are at the same location. Those skilled in the art will appreciate that many combinations are possible and all such combinations may be employed in this invention.

As described in the Applicant's prior patent specifications as summarised above, in alternative embodiments respective pairs of transmitting and receiving coils may be separated by a known fixed distance along the flow direction of the pipe. Each pair of transmitting and receiving coils may be operated as described above and can provide independent meshes or images of the flow at two points along the pipe. It is possible to cross-correlate the measurements from two transmitter/receiver pairs, in order to establish the time-of-flight of features that represent different phases in the multiphase flow. The time difference between the features provides the time it takes for the phase to travel over the fixed distance. Those skilled in the art will appreciate that the velocity of this phase is readily computed from this information. Correspondingly, a velocity profile across the cross section of the pipe may be obtained. That is, a mesh or image of velocities can be produced that can be used to establish the velocity differences between the primary/continuous phase, and any other phase. Such velocities can be obtained when the primary or continuous phase is either conducting (e.g. water) or non-conducting fluid (e.g. oil).

The electromagnetic measurement as described above can provide phase measurements where there is a conductivity contrast between the phases. This is possible when the different phases or constituents are flowing in a predominately conducting (e.g. water) or non-conducting (e.g. oil) primary phase.

Furthermore, alternative embodiments of the monitoring apparatus of the present invention for monitoring a multiphase flow in a pipe using magnetic induction tomography may comprise a plurality of, for example two, annular arrays of the transmitter/receiver coils 10, 12 disposed around the pipe 4 which defines therein an imaging space. In the first array, each first coil 10 is adapted to transmit an electromagnetic field when energized by an input electrical signal, and in the second array each second coil 12 is adapted to receive an electromagnetic field and generate an output electrical signal.

Preferably, each first coil 10 is circumferentially offset in a direction around the pipe 4, with respect to a respective adjacent second coil 12, to reduce or minimise direct electromagnetic coupling between the respective first and second coils 10, 12.

In some preferred embodiments, the first and second coils 10, 12 are each provided on a respective sheet of electrically insulating material, and the respective first or second coil 10, 12 and the sheet of electrically insulating material comprises a flexible printed circuit board.

As described in the Applicant's prior patent specifications, the electrical current may be selectively switched through selected first coils 10 in an array to generate from each energized coil a local electromagnetic field. Typically, an impedance (not shown) connected to at least some of the respective selected coils in the array is provided to modify the magnitude of the local electromagnetic field generated from the respective energized coil element.

In one embodiment, the electrical current may be selectively switched through selected coils in the array to provide a composite electromagnetic field generated from the energized first coils 10, when transmitting, and the energized first coils 10 have a controllable focal point within the pipe 4.

In another embodiment, the electrical current may be selectively switched through selected first coils 10 in the array 10 to provide a composite electromagnetic field received by the second coils 12, when receiving, from a controllable focal point within the pipe 4. The controllable focal point may be scanned across the array of first and second coils 10, 12 to scan the generated electromagnetic field across a cross-section of the pipe 4 and/or along a flow direction along the pipe 4. The scanning of the controllable focal point may be across a plurality of points to provide a pixelated image of the multiphase flow.

The present invention will now be described further with reference to the following nonlimiting Examples.

Example 1

Using the definitions for the phase sensitivity and water volume flowrate fraction as described above, the phase sensitivity of a planar, square coil as used in the monitoring apparatus of the present invention to provide transmitting and receiving coils was calculated theoretically, using simulations. The simulations were carried out when the coil was driven by 10MHz AC current.

A number of calculations of the phase sensitivity of the planar coil for a progressively increasing number of turns was made, and the results are shown in Figure 4. Calculations were made for the planar, square coil having 2, 6, 10, 14, 18 and 22 turns.

The value of the phase sensitivity was normalised to a normalised value within the range of from 0 to 1, with the value of 1 being the highest, and most desired, value. When the calculated parameter lies within a range, the normalisation is performed on the difference of the value relative to the limits of the range.

It can be seen from Figure 4 that for Example 1, the phase sensitivity was high, and when at least 6 turns are present the phase sensitivity achieves a normalised value of 1.

Furthermore, the phase dynamic range, as defined above, of the planar coil for a progressively increasing number of turns was also calculated, and the results are shown in Figure 5. Again, the value of the phase dynamic range was normalised as described above.

It can be seen from Figure 5 that for Example 1, the phase dynamic range was high, and when at least 6 turns are present the phase dynamic range achieves a normalised value of 1.

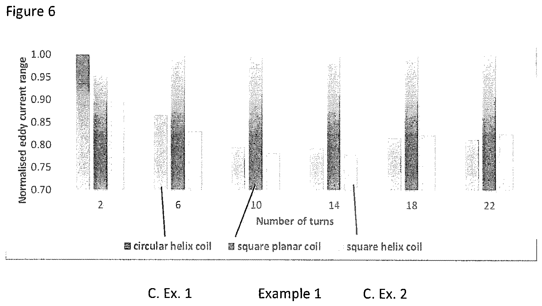

Finally, the eddy current range, as defined above, of the planar coil for a progressively increasing number of turns was also calculated, and the results are shown in Figure 6. Again, the value of the eddy current range was normalised as described above.

It can be seen from Figure 6 that for Example 1, the eddy current range was high, and when at least 6 turns are present the eddy current range achieves a normalised value of 1.

The Example was repeated using a rectangular, but non-square, planar coil, and it was found that enhanced measurement results were achieved up to a length and width coefficient of 2:1.

The Example was repeated using a different driving frequency. It was also found that at a low driving frequency of 2 kHz, which is a typical operating frequency for using MIT for imaging highly conductive objects, such as metals, pipelines, etc, the high normalised values for the phase sensitivity, the phase dynamic range and the eddy current range, were still achieved.

Comparative Example 1

Example 1 was repeated, but using a circular helical coil for the calculations, and the normalised values, for the progressively increasing number of turns, of phase sensitivity, phase dynamic range and eddy current were calculated and the results are shown in Figures 4 to 6.

As compared to Example 1, it can be seen that for 2 turns the normalised phase sensitivity, the normalised phase dynamic range and the normalised eddy current range for Comparative Example 1 are higher than for Example 1, but that as the number of turns increases, these parametric values for the circular helical coil are all lower than for Example 1. It is to be noted that a larger number of turns, i.e. more than 2 turns, is required to achieve the combination of high measuring sensitivity and measuring accuracy.

Comparative Example 2

Example 1 was repeated, but using a square helical coil for the calculations, and the normalised values, for the progressively increasing number of turns, of phase sensitivity, phase dynamic range and eddy current were calculated and the results are shown in Figures 4 to 6.

As compared to Example 1, it can be seen that for all of the measured number of turns, the normalised phase sensitivity, the normalised phase dynamic range and the normalised eddy current range for Comparative Example 2 are lower than for Example 1.

A comparison between the data of Example 1 and Comparative Examples 1 and 2, highlighted in Figures 4 to 6, shows that the planar and polygonal coil configuration used in the preferred embodiments of the present invention can provide the following advantages: a higher phase sensitivity, a larger phase dynamic range and greater induced eddy currents in the region of interest within a pipe carrying a multiphase flow.

Another advantage of the invention is from a mechanical point of view. The arrangement of a plurality of consecutive turns in a single plane avoids the tendency of the coil to depart from the pipe surface in proportion to the number of turns. Therefore, the proposed coil geometry reduces the radial dimension of the MIT apparatus mounted on or to the pipe.

It was also unexpected that the results of Figures 4 to 6 evidence that the trend for the self-resonance frequency of rectangular coils (1:1 ratio) shows a lower slope as compared to the trend from other geometries. In other words, for the planar and polygonal coil configuration used in the preferred embodiments of the present invention, the values of the phase sensitivity, phase dynamic range and induced eddy currents in the region of interest within a pipe carrying a multiphase flow do not significantly vary when the number of turns in the coil varies. This technical finding results in the possibility of utilising planar and polygonal coils with higher numbers of turns without experiencing instabilities associated with capacitance resonance.

Various other embodiments of the monitoring apparatus and method of the present invention within the scope of the appended claims will readily be apparent to those skilled in the art.

**Claims**

1. A monitoring apparatus (2) for monitoring a multiphase flow (8) in a pipe using magnetic induction tomography, the apparatus comprising a pipe (4) defining a flow conduit (6), at least one pair of first and second coils (10, 12) disposed on the pipe, wherein the first coil (10) is adapted to transmit an electromagnetic field into the flow conduit when energized by an input electrical signal and the second coil is adapted to receive an electromagnetic field from the flow conduit and generate an output electrical signal, wherein each of the first and second coils comprises an electrically conductive element (26) which is wound about a central axis (Z) to form a plurality of turns (28), the apparatus further comprising a controller (18) for selectively switching alternating electrical current through the first coil to generate from the energized first coil a local electromagnetic field which is transmitted into the fluid conduit, and for receiving an induced alternating electrical current from the second coil which is generated from the local electromagnetic field, **characterized in that** in the first and second coils (10, 12), each turn (28) is in a common plane (XY) so that the respective coil is planar and each turn is composed of a plurality of linear sections (30) and the respective coil is polygonal in plan; in the first and second coils (10, 12), the electrically conductive element (26) comprises an electrically conductive wire core (50) surrounded by an electrically non-conductive outer layer (52); in the first and second coils (10, 12), successive turns (28) are mutually adjacent and in contact, and adjacent turns of the electrically conductive element are mutually separated by an electrically insulating material.

2. A monitoring apparatus (2) according to claim 1 wherein each turn (28) is composed of four linear sections (42, 44, 46, 48) and the respective coil (10, 12) is rectangular in plan, optionally wherein the rectangular coil has a length: width ratio of from 1:1 to 3:1, further optionally wherein the rectangular coil has a length: width ratio of from 1: 1 to 2:1.

3. A monitoring apparatus (2) according to claim 1 or claim 2 wherein in each turn (28) the linear sections (30, 42, 44, 46, 48) are equal in length and the respective coil (10, 12) is a regular polygon in plan, optionally wherein the respective coil is square in plan.

4. A monitoring apparatus (2) according to any one of claims 1 to 3 wherein: i) the first and second coils (10, 12) are disposed on respective diametrically opposite first and second sides (14, 16) of the pipe (4); and/or ii) the central axis (Z) of the first coil (10) is coaxial with the central axis of the second coil (12).

5. A monitoring apparatus (2) according to any one of claims 1 to 4 wherein the electrically conductive wire core is composed of copper, further optionally wherein the core has a diameter of from 0.2 to 1 mm, yet further optionally wherein the core has a diameter of from 0.25 to 0.75 mm.

6. A monitoring apparatus (2) according to claim 5 wherein the electrically non-conductive outer layer (52) is composed of an enamel, optionally wherein the enamel outer layer has a thickness 0.02 to 0.04 mm.

7. A monitoring apparatus (2) according to claim 5 or claim 6 wherein in the first and second coils, successive wire turns are mutually adjacent and in contact along the entire length of each turn.

8. A monitoring apparatus (2) according to any one of claims 1 to 7 wherein: i) each of the first and second coils (10, 12) has from 10 to 30 turns, optionally wherein each of the first and second coils has from 15 to 25 turns; and/or ii) each of the first and second coils has an external dimension, in the plane (XY) of the respective coil, within the range of from 20 to 50 mm, optionally wherein each of the first and second coils has an external dimension, in the plane of the respective coil, within the range of from 25 to 45 mm; and/or iii) each of the first and second coils has an external dimension, in the plane of the respective coil, relative to a diameter (D) of the pipe (4) within the range of from 0.1D to 0.8D, optionally wherein each of the first and second coils has an external dimension, in the plane of the respective coil, relative to a diameter (D) of the pipe within the range of from 0.2D to 0.4D.

9. A method of monitoring a multiphase flow in a pipe using magnetic induction tomography, the method comprising the steps of:

a. providing a pipe (4) defining a flow conduit (6), and at least one pair of first and second coils (10, 12) disposed on the pipe, each of the first and second coils being adapted to transmit an electromagnetic field into the flow conduit when energized by an input electrical signal and/or to receive an electromagnetic field from the flow conduit and generate an output electrical signal, wherein each of the first and second coils comprises an electrically conductive element (26) which is wound about a central axis (Z) to form a plurality of turns (28);
b. flowing a multiphase flow (8) along the pipe;
c. transmitting an electromagnetic field from the first coil (10) into the multiphase flow; and
d. receiving by the second coil (12) an electromagnetic field from the multiphase flow and generating an output electrical signal therefrom, **characterized in that** in the first and second coils (10, 12), each turn (28) is in a common plane (XY) so that the respective coil is planar and each turn is composed of a plurality of linear sections (30) and the respective coil is polygonal in plan; in the first and second coils (10, 12), the electrically conductive element (26) comprises an electrically conductive wire core (50) surrounded by an electrically non-conductive outer layer (52); in the first and second coils, successive turns (28) are mutually adjacent and in contact, and adjacent turns of the electrically conductive element are mutually separated by an electrically insulating material.

10. A method according to claim 9 wherein each turn (28) is composed of four linear sections (42, 44, 46, 48) and the respective coil is rectangular in plan, optionally wherein the rectangular coil has a length: width ratio of from 1:1 to 3:1, further optionally wherein the rectangular coil has a length: width ratio of from 1:1 to 2:1.

11. A method according to claim 9 or claim 10 wherein in each turn (28) the linear sections (30, 42, 44, 46, 48) are equal in length and the respective coil (10, 12) is a regular polygon in plan, optionally wherein the respective coil is square in plan.

12. A method according to any one of claims 9 to 11 wherein the first and second coils (10, 12) are disposed on respective diametrically opposite first and second sides (14, 16) of the pipe (4), optionally wherein the central axis (Z) of the first coil (10) is coaxial with the central axis of the second coil (12).

13. A method according to any one of claims 9 to 12 wherein in the first and second coils, successive turns are mutually adjacent and in contact along the entire length of each turn.

14. A method according to any one of claims 9 to 13 wherein: i) each of the first and second coils (10, 12) has from 10 to 30 turns, optionally wherein each of the first and second coils has from 15 to 25 turns; and/or ii) in step c alternating electrical current having a frequency of from 1 kHz to 30 MHz is switched through the first coil (10) to generate from the respective energized coil a local electromagnetic field which is transmitted into the fluid conduit (6), optionally wherein in step c the alternating electrical current has a frequency of from 1 kHz to 10 MHz.

15. A method according to any one of claims 9 to 14 wherein the multiphase flow (8) comprises at least two phases selected from an oil phase, an aqueous phase, a solid phase and a gaseous phase, optionally wherein the multiphase flow comprises at least three phases selected from an oil phase, an aqueous phase, a solid phase and a gaseous phase, further optionally wherein the multiphase flow comprises all of the phases selected from an oil phase, an aqueous phase, a solid phase and a gaseous phase.

**Patentansprüche**

1. Überwachungsvorrichtung (2) zum Überwachen einer Mehrphasenströmung (8) in einem Rohr unter Verwendung von magnetischer Induktionstomographie, wobei die Vorrichtung ein Rohr (4) aufweist, das eine Strömungsleitung (6) definiert, und wenigstens ein Paar von ersten und zweiten Spulen (10, 12), die auf dem Rohr angeordnet sind, wobei die erste Spule (10) angepasst ist, um ein elektromagnetisches Feld in die Strömungsleitung zu übertragen, wenn sie durch ein elektrisches Eingangssignal erregt wird, und die zweite Spule angepasst ist, um ein elektromagnetisches Feld von der Strömungsleitung zu empfangen und ein elektrisches Ausgangssignal zu erzeugen, wobei jede der ersten und zweiten Spulen ein elektrisch leitendes Element (26) aufweist, das um eine Mittelachse (Z) gewickelt ist, um mehrere Windungen (28) zu bilden, wobei die Vorrichtung ferner eine Steuerung (18) zum selektiven Schalten von elektrischem Wechselstrom durch die erste Spule aufweist, um von der erregten ersten Spule ein lokales elektromagnetisches Feld zu erzeugen, das in die Fluidleitung übertragen wird, und zum Empfangen eines induzierten elektrischen Wechselstroms von der zweiten Spule, der von dem lokalen elektromagnetischen Feld erzeugt wird, **dadurch gekennzeichnet, dass** in der ersten und zweiten Spule (10, 12) jede Windung (28) in einer gemeinsamen Ebene (XY) ist, sodass die jeweilige Spule planar ist und jede Windung aus mehreren linearen Abschnitten (30) besteht und die jeweilige Spule im Grundriss mehreckig ist; in der ersten und zweiten Spulen (10, 12) das elektrisch leitende Element (26) einen elektrisch leitenden Drahtkern (50) aufweist, der von einer elektrisch nicht leitenden Außenschicht (52) umgeben ist; in der ersten und zweiten Spule (10, 12) aufeinanderfolgende Windungen (28) aneinandergrenzen und in Kontakt sind und benachbarte Windungen des elektrisch leitenden Elements durch ein elektrisch isolierendes Material voneinander getrennt sind.

2. Überwachungsvorrichtung (2) nach Anspruch 1, wobei jede Windung (28) aus vier linearen Abschnitten (42, 44, 46, 48) besteht und die jeweilige Spule (10, 12) im Grundriss rechteckig ist, wobei optional die rechteckige Spule ein Längen-Breiten-Verhältnis von 1:1 bis 3:1 hat, wobei ferner optional die rechteckige Spule ein Längen-Breiten-Verhältnis von 1:1 bis 2:1 hat.

3. Überwachungsvorrichtung (2) nach Anspruch 1 oder Anspruch 2, wobei in jeder Windung (28) die linearen Abschnitte (30, 42, 44, 46, 48) gleich lang sind und die jeweilige Spule (10, 12) im Grundriss ein regelmäßiges Vieleck ist, wobei optional die jeweilige Spule im Grundriss quadratisch ist.

4. Überwachungsvorrichtung (2) nach einem der Ansprüche 1 bis 3, wobei: i) die erste und zweite Spule (10, 12) auf jeweiligen diametral gegenüberliegenden ersten und zweiten Seiten (14, 16) des Rohrs (4) angeordnet sind; und/oder ii) die Mittelachse (Z) der ersten Spule (10) koaxial mit der Mittelachse der zweiten Spule (12) ist.

5. Überwachungsvorrichtung (2) nach einem der Ansprüche 1 bis 4, wobei der elektrisch leitende Drahtkern aus Kupfer besteht, wobei ferner optional der Kern einen Durchmesser von 0,2 bis 1 mm hat, und wobei ferner optional der Kern einen Durchmesser von 0,25 bis 0,75 mm hat.

6. Überwachungsvorrichtung (2) nach Anspruch 5, wobei die elektrisch nicht-leitende Außenschicht (52) aus einem Email besteht, wobei optional die Email-Außenschicht eine Dicke von 0,02 bis 0,04 mm hat.

7. Überwachungsvorrichtung (2) nach Anspruch 5 oder Anspruch 6, wobei in der ersten und zweiten Spule aufeinanderfolgende Drahtwindungen aneinander angrenzend und entlang der gesamten Länge jeder Windung in Kontakt sind.

8. Überwachungsvorrichtung (2) nach einem der Ansprüche 1 bis 7, wobei: i) jede der ersten und zweiten Spulen (10, 12) 10 bis 30 Windungen hat, wobei optional jede der ersten und zweiten Spulen 15 bis 25 Windungen hat; und/oder ii) jede der ersten und zweiten Spulen eine Außenabmessung in der Ebene (XY) der jeweiligen Spule im Bereich von 20 bis 50 mm hat, wobei optional jede der ersten und zweiten Spulen eine Außenabmessung in der Ebene der jeweiligen Spule im Bereich von 25 bis 45 mm hat; und/oder iii) jede der ersten und zweiten Spulen eine Außenabmessung in der Ebene der jeweiligen Spule relativ zu einem Durchmesser (D) des Rohrs (4) im Bereich von 0,1D bis 0,8D hat, wobei optional jede der ersten und zweiten Spulen eine Außenabmessung in der Ebene der jeweiligen Spule relativ zu einem Durchmesser (D) des Rohrs im Bereich von 0,2D bis 04D hat.

9. Verfahren zum Überwachen einer Mehrphasenströmung in einem Rohr unter Verwendung von magnetischer Induktionstomographie, wobei das Verfahren diese Schritte aufweist:

a. Bereitstellen eines Rohrs (4), das eine Strömungsleitung (6) definiert, und wenigstens eines Paars von ersten und zweiten Spulen (10, 12), die auf dem Rohr angeordnet sind, wobei jede der ersten und zweiten Spulen angepasst ist, um ein elektromagnetisches Feld in die Strömungsleitung zu übertragen, wenn sie durch ein elektrisches Eingangssignal erregt wird, und/oder ein elektromagnetisches Feld von der Strömungsleitung zu empfangen und ein elektrisches Ausgangssignal zu erzeugen, wobei jede der ersten und zweiten Spulen ein elektrisch leitfähiges Element (26) aufweist, das um eine Mittelachse (Z) gewickelt ist, um mehrere Windungen (28) zu bilden;

b. Strömen eines Mehrphasenstroms (8) entlang des Rohres;

c. Übertragen eines elektromagnetischen Feldes von der ersten Spule (10) in den Mehrphasenstrom; und

d. Empfangen eines elektromagnetischen Feldes von der Mehrphasenströmung durch die zweite Spule (12) und Erzeugen eines elektrischen Ausgangssignals daraus, **dadurch gekennzeichnet, dass** in der ersten und der zweiten Spule (10, 12) jede Windung (28) in einer gemeinsamen Ebene (XY) ist, sodass die jeweilige Spule planar ist und jede Windung aus mehreren linearen Abschnitten (30) besteht und die jeweilige Spule im Grundriss mehreckig ist; in der ersten und zweiten Spule (10, 12) das elektrisch leitende Element (26) einen elektrisch leitenden Drahtkern (50) aufweist, der von einer elektrisch nicht leitenden Außenschicht (52) umgeben ist; in der ersten und zweiten Spule aufeinanderfolgende Windungen (28) aneinandergrenzen und in Kontakt sind und benachbarte Windungen des elektrisch leitenden Elements durch ein elektrisch isolierendes Material voneinander getrennt sind.

10. Verfahren nach Anspruch 9, wobei jede Windung (28) aus vier linearen Abschnitten (42, 44, 46, 48) besteht und die jeweilige Spule im Grundriss rechteckig ist, wobei optional die rechteckige Spule ein Längen-Breiten-Verhältnis von 1:1 bis 3:1 hat, wobei ferner optional die rechteckige Spule ein Längen-Breiten-Verhältnis von 1:1 bis 2:1 hat.

11. Verfahren nach Anspruch 9 oder Anspruch 10, wobei in jeder Windung (28) die linearen Abschnitte (30, 42, 44, 46, 48) gleich lang sind und die jeweilige Spule (10, 12) im Grundriss ein regelmäßiges Vieleck ist, wobei optional die jeweilige Spule im Grundriss quadratisch ist.

12. Verfahren nach einem der Ansprüche 9 bis 11, wobei die erste und zweite Spule (10, 12) auf jeweiligen diametral gegenüberliegenden ersten und zweiten Seiten (14, 16) des Rohrs (4) angeordnet sind, wobei optional die Mittelachse (Z) der ersten Spule (10) koaxial mit der Mittelachse der zweiten Spule (12) ist.

13. Verfahren nach einem der Ansprüche 9 bis 12, wobei in der ersten und zweiten Spule aufeinanderfolgende Windungen aneinander angrenzend und entlang der gesamten Länge jeder Windung in Kontakt sind.

**14.** Verfahren nach einem der Ansprüche 9 bis 13, wobei: i) jede der ersten und zweiten Spulen (10, 12) 10 bis 30 Windungen hat, wobei optional jede der ersten und zweiten Spulen 15 bis 25 Windungen hat; und/oder ii) in Schritt c elektrischer Wechselstrom mit einer Frequenz von 1 kHz bis 30 MHz durch die erste Spule (10) geschaltet wird, um aus der jeweiligen erregten Spule ein lokales elektromagnetisches Feld zu erzeugen, das in die Fluidleitung (6) übertragen wird, wobei optional in Schritt c der elektrische Wechselstrom eine Frequenz von 1 kHz bis 10 MHz hat.

**15.** Verfahren nach einem der Ansprüche 9 bis 14, wobei der Mehrphasenstrom (8) wenigstens zwei Phasen aufweist, die aus einer Ölphase, einer wässrigen Phase, einer festen Phase und einer gasförmigen Phase ausgewählt sind, wobei optional der Mehrphasenstrom wenigstens drei Phasen aufweist, die aus einer Ölphase, einer wässrigen Phase, einer festen Phase und einer gasförmigen Phase ausgewählt sind, wobei ferner optional der Mehrphasenstrom alle Phasen aufweist, die aus einer Ölphase, einer wässrigen Phase, einer festen Phase und einer gasförmigen Phase ausgewählt sind.

**Revendications**

**1.** Appareil de surveillance (2) destiné à surveiller un écoulement polyphasique (8) dans un tuyau à l'aide de la tomographie par induction magnétique, l'appareil comprenant un tuyau (4) définissant un conduit d'écoulement (6), au moins une paire de première et deuxième bobines (10, 12) disposées sur le tuyau, dans lequel la première bobine (10) est adaptée pour transmettre un champ électromagnétique dans le conduit d'écoulement lorsqu'elle est excitée par un signal électrique d'entrée et la deuxième bobine est adaptée pour recevoir un champ électromagnétique provenant du conduit d'écoulement et générer un signal électrique de sortie, dans lequel chacune des première et deuxième bobines comprend un élément électriquement conducteur (26) qui est enroulé autour d'un axe central (Z) pour former une pluralité de spires (28), l'appareil comprenant en outre un contrôleur (18) servant à commuter sélectivement un courant électrique alternatif à travers la première bobine pour générer, à partir de la première bobine excitée, un champ électromagnétique local qui est transmis dans le conduit de fluide, et servant à recevoir un courant électrique alternatif induit provenant de la deuxième bobine qui est généré à partir du champ électromagnétique local, **caractérisé en ce que** :

dans les première et deuxième bobines (10, 12), chaque spire (28) est dans un plan commun (XY) de sorte que la bobine respective est plane et chaque spire est composée d'une pluralité de sections linéaires (30) et la bobine respective est polygonale en plan ;
dans les première et deuxième bobines (10, 12), l'élément électriquement conducteur (26) comprend une âme de fil électriquement conductrice (50) qui est entourée d'une couche externe électriquement non conductrice (52) ;
dans les première et deuxième bobines (10, 12), des spires successives (28) sont mutuellement adjacentes et en contact, et des spires adjacentes de l'élément électriquement conducteur sont mutuellement séparées par un matériau électriquement isolant.

**2.** Appareil de surveillance (2) selon la revendication 1, dans lequel chaque spire (28) est composée de quatre sections linéaires (42, 44, 46, 48) et la bobine respective (10, 12) est rectangulaire en plan, éventuellement dans lequel la bobine rectangulaire a un rapport de longueur à largeur allant de 1 : 1 à 3 : 1, en outre éventuellement dans lequel la bobine rectangulaire a un rapport de longueur à largeur allant de 1 : 1 à 2 : 1.

**3.** Appareil de surveillance (2) selon la revendication 1 ou 2, dans lequel, dans chaque spire (28), les sections linéaires (30, 42, 44, 46, 48) sont de longueur égale et la bobine respective (10, 12) est un polygone régulier en plan, éventuellement dans lequel la bobine respective est carrée en plan.

**4.** Appareil de surveillance (2) selon l'une quelconque des revendications 1 à 3, dans lequel : i) les première et deuxième bobines (10, 12) sont disposées sur de respectifs premier et deuxième côtés diamétralement opposés (14, 16) du tuyau (4) ; et/ou ii) l'axe central (Z) de la première bobine (10) et l'axe central de la deuxième bobine (12) sont coaxiaux.

**5.** Appareil de surveillance (2) selon l'une quelconque des revendications 1 à 4, dans lequel l'âme de fil électriquement conductrice est composée de cuivre, en outre éventuellement dans lequel l'âme a un diamètre allant de 0,2 à 1 mm, encore en outre éventuellement dans lequel l'âme a un diamètre allant de 0,25 à 0,75 mm.

**6.** Appareil de surveillance (2) selon la revendication 5, dans lequel la couche externe électriquement non conductrice (52) est composée d'un émail, éventuellement dans lequel la couche externe d'émail a une épaisseur allant de 0,02 à 0,04 mm.

**7.** Appareil de surveillance (2) selon la revendication 5 ou 6, dans lequel, dans les première et deuxième bobines, des spires de fil successives sont mutuellement adjacentes et en contact sur toute la longueur de chaque spire.

8. Appareil de surveillance (2) selon l'une quelconque des revendications 1 à 7, dans lequel : i) chacune des première et deuxième bobines (10, 12) comporte de 10 à 30 spires, éventuellement dans lequel chacune des première et deuxième spires comporte de 15 à 25 spires ; et/ou ii) chacune des première et deuxième bobines a une dimension externe, dans le plan (XY) de la bobine respective, comprise dans la plage allant de 20 à 50 mm, éventuellement dans lequel chacune des première et deuxième bobines a une dimension externe, dans le plan de la bobine respective, comprise dans la plage allant de 25 à 45 mm ; et/ou iii) chacune des première et deuxième bobines a une dimension externe, dans le plan de la bobine respective, par rapport à un diamètre (D) du tuyau (4), comprise dans la plage allant de 0,1D à 0,8D, éventuellement dans lequel chacune des première et deuxième bobines a une dimension externe, dans le plan de la bobine respective, par rapport à un diamètre (D) du tuyau, comprise dans la plage allant de 0,2D à 0,4D.

9. Procédé de surveillance d'un écoulement polyphasique dans un tuyau à l'aide de la tomographie par induction magnétique, le procédé comprenant les étapes consistant à :

a. fournir un tuyau (4) définissant un conduit d'écoulement (6), et au moins une paire de première et deuxième bobines (10, 12) disposées sur le tuyau, chacune des première et deuxième bobines étant adaptée pour transmettre un champ électromagnétique dans le conduit d'écoulement lorsqu'elle est excitée par un signal électrique d'entrée, et/ ou pour recevoir un champ électromagnétique provenant du conduit d'écoulement et générer un signal électrique de sortie, dans lequel chacune des première et deuxième bobines comprend un élément électriquement conducteur (26) qui est enroulé autour d'un axe central (Z) pour former une pluralité de spires (28) ;
b. faire circuler un écoulement polyphasique (8) le long du tuyau ;
c. transmettre un champ électromagnétique provenant de la première bobine (10) dans l'écoulement polyphasique ; et
d. recevoir, par la deuxième bobine (12), un champ électromagnétique provenant du flux polyphasique et générer un signal électrique de sortie à partir de celui-ci,

**caractérisé en ce que** :

dans les première et deuxième bobines (10, 12), chaque spire (28) est dans un plan commun (XY) de sorte que la bobine respective est plane et chaque spire est composée d'une pluralité de sections linéaires (30) et la bobine respective est polygonale en plan ;
dans les première et deuxième bobines (10, 12), l'élément électriquement conducteur (26) comprend une âme de fil électriquement conductrice (50) qui est entourée d'une couche externe électriquement non conductrice (52) ;
dans les première et deuxième bobines, des spires successives (28) sont mutuellement adjacentes et en contact, et des spires adjacentes de l'élément électriquement conducteur sont mutuellement séparées par un matériau électriquement isolant.

10. Procédé selon la revendication 9, dans lequel chaque spire (28) est composée de quatre sections linéaires (42, 44, 46, 48) et la bobine respective est rectangulaire en plan, éventuellement dans lequel la bobine rectangulaire a un rapport de longueur à largeur allant de 1 : 1 à 3 : 1, en outre éventuellement dans lequel la bobine rectangulaire a un rapport de longueur à largeur allant de 1 : 1 à 2 : 1.

11. Procédé selon la revendication 9 ou 10, dans lequel, dans chaque spire (28), les sections linéaires (30, 42, 44, 46, 48) sont de longueur égale et la bobine respective (10, 12) est un polygone régulier en plan, éventuellement dans lequel la bobine respective est carrée en plan.

12. Procédé selon l'une quelconque des revendications 9 à 11, dans lequel les première et deuxième bobines (10, 12) sont disposées sur de respectifs premier et deuxième côtés diamétralement opposés (14, 16) du tuyau (4), éventuellement dans lequel l'axe central (Z) de la première bobine (10) et l'axe central de la deuxième bobine (12) sont coaxiaux.

13. Procédé selon l'une quelconque des revendications 9 à 12, dans lequel, dans les première et deuxième bobines, des spires successives sont mutuellement adjacentes et en contact sur toute la longueur de chaque spire.

14. Procédé selon l'une quelconque des revendications 9 à 13, dans lequel : i) chacune des première et deuxième bobines (10, 12) comporte de 10 à 30 spires, éventuellement dans lequel chacune des première et deuxième bobines comporte de 15 à 25 spires ; et/ou ii) à l'étape c, un courant électrique alternatif ayant une fréquence allant de 1 kHz à 30 MHz est commuté à travers la première bobine (10) pour générer, à partir de la bobine excitée respective, un champ électromagnétique local qui est transmis dans le conduit de fluide (6), éventuellement dans lequel, à l'étape c, le courant électrique alternatif a une fréquence allant de 1 kHz à 10 MHz.

15. Procédé selon l'une quelconque des revendications 9 à 14, dans lequel l'écoulement polyphasique (8) comprend au moins deux phases sélectionnées parmi une phase huileuse, une phase aqueuse, une phase solide et une phase gazeuse, éventuellement dans lequel l'écoulement polyphasique comprend au moins trois phases sélectionnées parmi une phase huileuse, une phase aqueuse, une phase solide et une phase gazeuse, en outre éventuellement dans lequel l'écoulement polyphasique comprend toutes les phases sélectionnées parmi une phase huileuse, une phase aqueuse, une phase solide et une phase gazeuse.

Figure 1

16  12                    20                                        18

2

Driver

Coil - Rx

8

Data Acquisition
System

Processor

Coil -Tx

6

4    14      10                                22                        24

Figure 2          10, 12  38    26    28    30

32          34

44      42    40    46    48

Figure 3    26        50            52

Figure 4

C. Ex. 1          Example 1        C. Ex. 2

Figure 5

C. Ex. 1          Example 1        C. Ex. 2

Figure 6

C. Ex. 1        Example 1        C. Ex. 2

**REFERENCES CITED IN THE DESCRIPTION**

**Patent documents cited in the description**

- GB 2513678 B
- GB 2513679 B
- GB 2507368 B
- GB 2534337 B
- GB 2530601 B
- GB 2527324 B
- CN 101871906 A